# EUROPEAN PATENT SPECIFICATION

(11) **EP 1 748 792 B1**
(45) Date of publication and mention of the grant of the patent: **11.02.2009**
(21) Application number: 05750379.9
(22) Date of filing: 26.05.2005
(51) Int. Cl.: A61K 39/39, A61K 31/70, A61P 35/00

(54) **COMPOSITIONS COMPRISING DEMETHYLATING AGENTS AS ENHANCERS OF IMMUNOTHERAPY FOR THE TREATMENT OF CHRONIC INFECTIONS AND NEOPLASTIC DISEASES AND METHODS FOR TREATING THE SAME**
ZUSAMMENSETZUNGEN AUS DEMETHYLIERENDEN MITTELN ALS VERSTÄRKER DER IMMUNTHERAPIE ZUR BEHANLDUNG VON CHRONISCHEN INFEKTIONEN UND NEOPLASTISCHEN ERKRANKUNGEN UND BEHANDLUNGSVERFAHREN DAFÜR
COMPOSITIONS COMPRENANT DES AGENTS DEMETHYLISANTS RENFORÇANT L'IMMUNOTHERAPIE DANS LE TRAITEMENT D'INFECTIONS CHRONIQUES ET DE MALADIES NEOPLASTIQUES, ET PROCEDE DE TRAITEMENT ASSOCIE

(30) Priority: 26.05.2004 US 574638 P; 24.06.2004 US 582455 P; 26.05.2005 US 138171
(43) Date of publication of application: 07.02.2007
(73) Proprietor: Biovaxim Limited, 5-7 Cranwood Street London EC1V 9EE (GB)
(72) Inventor: ANDRIEU, Jean-Marie, F-75005 Paris (FR); WEI-LU, Louis, F-75015 Paris (FR)
(74) Representative: Breese, Pierre
(86) International application number: PCT/IB2005/001827
(87) International publication number: WO 2005/115450

(56) References cited:
- WO-A-95/28940
- TODD S. WEISER ET AL.: "Sequential 5-aza-2'-deoxycytidine-depsipeptide FR901228 treatment induces apoptosis preferentially in cancer cells and facilitates recognition by cytotoxic T lymphocytes specific for NY-ESO-1" JOURNAL OF IMMUNOTHERAPY, vol. 24, no. 2, 2001, pages 151-161, XP008053516
- SERRANO ALFONSO ET AL: "Expression of HLA class I antigens and restoration of antigen-specific CTL response in melanoma cells following 5-Aza-2'-deoxycytidine treatment" INTERNATIONAL JOURNAL OF CANCER, vol. 94, no. 2, 15 October 2001 (2001-10-15), pages 243-251, XP002348088 ISSN: 0020-7136
- MARIA G. MASUCCI ET AL.: "5-azacytidine up regulates the expression of Epstein-Barr virus nuclear antigen 2 (EBNA-2) through EBNA-6 and latent membrane protein in the Burkitt's lymphoma line Rael" JOURNAL OF VIROLOGY, vol. 63, no. 7, July 1989 (1989-07), pages 3135-3141, XP002348089

## Description

### BACKGROUND OF THE INVENTION

Vaccinal compositions are of great importance for human health. Due to the variety of diseases, said compositions are not still able to induce a sufficient immune response in order to prevent or treat the associated disease. In the prior art, adjuvants have been developed in order to enhance the specific immune response induced. Said adjuvants enable the obtaining of a sufficient immune response to prevent or treat the associated diseases. Example of such new adjuvants include immunostimulatory oligonucleotides like CpG nucleotides. Nevertheless, in the case of tumor diseases or chronic virus diseases, the immune response obtained with such compositions is sometimes still insufficient in order to treat them.

So, there is a recognized and permanent need in the art for new compositions and methods for enhancing *in vivo* antigen-specific immune response in a subject.

The purpose of the present invention is to fulfil this need by providing new compositions for enhancing such an antigen-specific immune response.

The importance of epigenetic mechanisms in the initiation and progression of human cancer has now been established (JONES and BAYLIN, Nat. Rev. Genet., vol.3, p: 415-28, 2002). Patterns of DNA methylation and chromatin structure are profoundly altered in neoplasia. These epigenetic changes (and particularly the aberrant promoter hypermethylation associated with inappropriate gene silencing) affect virtually every step in tumour progression. The finding that many genes controlling normal cellular homeostasis can be silenced inappropriately by structural chromatin changes that involve DNA methylation has encouraged a search for agents that might reverse these changes. So far, two classes of DNA methylating agents have been identified: the first one includes artificially synthesized nucleotides which, once incorporated into the nucleic acids of dividing cells serve as competitive inhibitors of DNA methyltransferase enzymes ; the other one is made of chemical compounds capable of non-competitively inhibiting the methyltransferases.

Among the first class of demethylating agents, 5-azacytidine and its deoxy derivative 5-aza-2'-deoxycytidine were first synthesized as potential chemotherapeutic agents for cancer (CIHAK, Oncology, vol.30, p: 405-22, 1974). These agents are powerful inhibitors of DNA methylation. Once they are incorporated into the nucleic acids of dividing cells, they act as inhibitors of DNA methyltransferases (JONES and TAYLOR, Cell, vol.20, p: 85-93, 1980). Their use for restoring gene function in treated cells in culture has suggested that they might be useful for treating patients with malignant diseases (LUBBERT, Curr. Top. Microbiol. Immunol., vol.249, p: 135-64, 2000). Numerous clinical trials have evaluated the efficacy of 5-azacitidine in hematologic malignancies. As a single agent, it was demonstrated to have a limited activity in relapsed or refractory acute myelogenous leukaemia (AML) and myolodysplastic syndrome (MDS). A number of phase II trials of single-agent azacitidine utilizing a variety of doses and schedules have also been performed in solid tumours, including breast, gastrointestinal, lung, kidney, and testicular cancers as well as melanoma, sarcoma, non-Hodgkin's and Hodgkin's lymphomas. Although occasional responses have been observed, results were uniformly disappointing, and no further large-scale studies were initiated (DOWELL and MINNA, J. Clin. Oncol., vol.22, p: 1353-5, 2004). A recent clinical trial conducted with Epstein-Barr virus (EBV)-associated tumors has demonstrated that 5-azacytidine reversed the dense CpG methylation of latent EBV promoters in tumor tissues (CHAN et al., J. Clin. Oncol., vol.22, p: 1373-81, 2004). Recently, new nucleotide analogue have been identified like zebularine (1-(beta-D-ribofuranosyl)-1,2dihydropropyrimindin-2-one; CHENG et al., Cancer Res., vol.95(5), p:399-409, 2003). Moreover the demethylating agent 5-aza-2'-deoxycytidine was shown to induce the expression of tumour antigen NY-ESO-1 upon which the cancer cells are recognized by CTLs specific for NY-ESO-1 and therefore induce apoptosis in said cancer cells (Todds et al., J. Immunotherapy, vol.24, p: 151-161, 2001), and to induce the expression of HlA class I tumour antigens allowing cancer cell recognition by MAGE specific CTLs (Serrano et al., Int. J. Cancer, vol.94, p: 243-251, 2001).

Among the second class of demethylating drugs, procanaimide (which is a drug used over the past 20 years for the treatment of cardiac arrhythmias) is a non-competitive inhibitor of methyltransferases. This compound is a derivative of 4-amino-benzoic acid -i.e. amide with 2-(diethylamino)ethylamine. It has now been shown to reactivate the P16 transcription from a methylation-silenced promoter in prostate cancer cells that grow in nude mice (LIN et al., Cancer Res., vol.61, p: 8611-6, 2001). Recently, a new derivative of 4-amino-benzoic acid with demethylating activity has been identified: Procaine (VILLAR-GAREA *et al.,* vol.63, p:4984-4989, 2003) corresponding to the ester of 4-amino-benzoic acid with 2-(diethylamino)ethanol.

One concern about the use of demethylating agents in patients is whether the drugs will cause an inappropriate gene activation in normal cells. Although important, this problem might not be insurmountable in as much as transcription from promoter-containing CpG islands is not commonly controlled by methylation in normal cells (WOLF and MIGEON, Nature, vol.295, p: 667-71, 1982; BIRD, Genes Dev., viol.16, p: 6-21, 2002).

Actually, the role of epigenetic mechanisms in the immune system fonctionning is in its first phase of exploration. There is now some evidence for the epigenetic regulation of the expression of cytokines such as IL-2 (RICHTER et al., J. Exp. Med., vol.190, p:1439-50, 1999; BRUNIQUEL and SCHWARTZ, Nat. Immunol. Vol.4, p: 235-40, 2003) or IFN-g (FITZPATRICK et al., J. Immunol., vol.162, p: 5053-7, 1999; PANEK et al., J. Immunol., vol.153, p: 4555-64, 1994; YANO et al., J. Immunol., vol.171, p: 2510-6, 2003). It has also been reported that DNA methyltransferase was upregulated in human immunodeficiency virus (HIV), infection resulting in the methylation of IFN-g promoter and the subsequent down-regulation of IFN-g expression (FANG et al., J. Virol., vol.75, p: 9753-61, 2001; MIKOVITS et al., Mol. Cell. Biol., vol.18, p: 5166-77, 1998).

Unexpectedly, the inventors have demonstrated that the administration of a demethylating agent -i.e. 5-azacytidine- potentiates the antigen-specific cytolysis by cytotoxic T lymphocytes (CTL) obtained after the administration of a specific antigen -i.e. a virus antigen-.

### SUMMARY OF THE INVENTION

One aspect of this invention provides for compositions comprising demethylating agents and virus antigens that are helpful in inducing and enhancing antigen-specific cytotoxic T lymphocytes (CTL or effectors) functions, preferably cytolysis by cytotoxic T lymphocytes (CTL) *in vivo,* which could be impaired in patients with chronic infections. Another aspect of this invention provides for kit comprising (i) a first composition comprising a demethylating agent and (ii) a second composition comprising a virus antigen. Such compositions and kits are suitable to induce and enhance virus-specific immunity to chronic viral diseases wherein antiviral CTL is present but not functionally activated. In yet another aspect of this invention, there is provides uses for administrating the above mentionned demethylating agents and specific antigens.

### BRIEF DESCRIPTION OF THE DRAWINGS

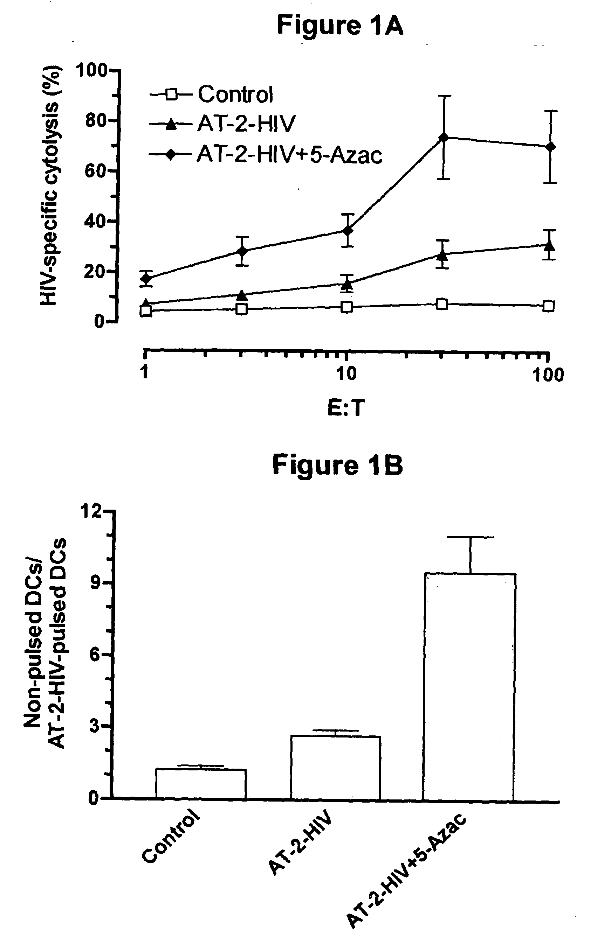

Fig. 1A shows *ex vivo* HIV-specific cytolysis of AT-2-HIV pulsed DCs by splenocytes was increased >2 fold in 5-Azacytidine-treated immunized animals as compared with untreated animals (P<0.01). These results show enhanced *ex vivo* HIV-specific cytolysis in 5-Azacytidine-treated with a therapeutically effective amount AT-2-HIV-immunized mice.
Fig. 1B shows *in vivo* HIV-specific cytolysis of AT-2-HIV-pulsed DCs was increased >3 fold in 5-Azacytidine-treated immunized animals as compared with untreated immunized animals (P<0.01). These results show enhanced *in vivo* HIV-specific cytolysis in 5-Azacytidine-treated with a therapeutically effective amount of AT-2-HIV-immunized mice.

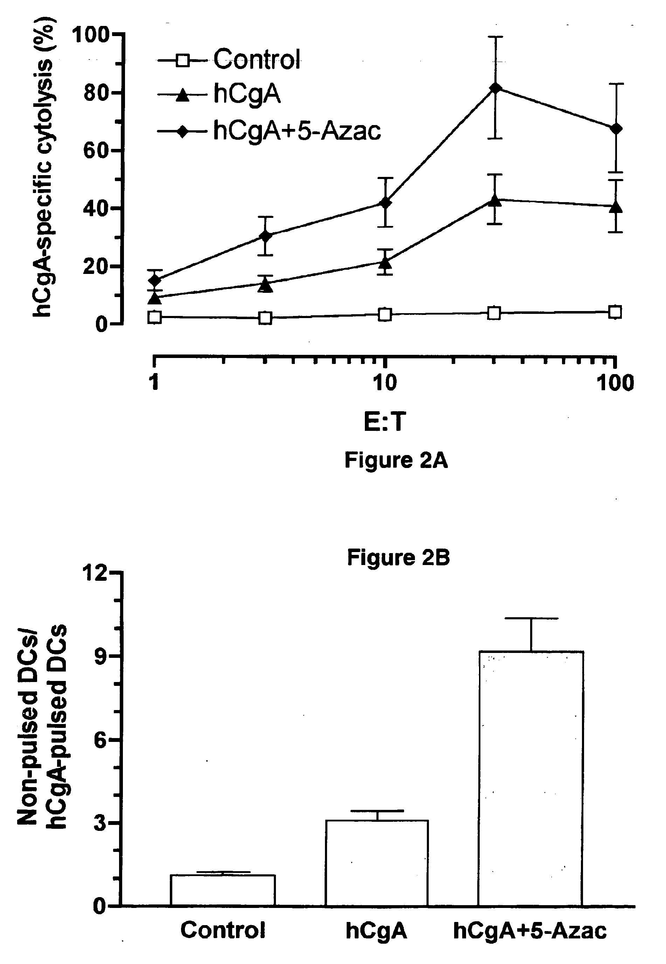

Fig. 2A shows *ex vivo* hCgA-specific cytolysis of hCgA-pulsed DCs by splenocytes was increased >2 fold in 5-Azacytidine-treated immunized animals as compared with untreated immunized animals (P<0.01). These results show enhanced *ex vivo* HIV-specific cytolysis in 5-Azacytidine-treated with a therapeutically effective amount of hCgA-immunized mice.
Fig. 2B shows *in vivo* hCgA-specific cytolysis of hCgA-pulsed by splenocytes was increased >3 fold in 5-Azacytidine-treated immunized animals as compared with untreated immunized animals (P<0.01). These results show enhanced *in vivo* hCgA-specific cytolysis in 5-Azacytidine-treated with a therapeutically effective amount hCgA-immunized mice.

### DETAILED DESCRIPTION OF THE INVENTION

In a first aspect the present invention relates to the use of a demethylating agent for the manufacture of a medicament for the prevention or treatment of chronic viral diseases wherein antiviral CTL are present but not-functionally activated, wherein said demethylating agent induces a virus specific immunity and said medicament enhances the *in vivo* antigen-specific cytolysis by CTL in a subject.

As used herein, the term "subject" denotes a Mammal, such as a rodent, a feline, a canine and a primate. The subject is an animal such as cow, pig, horse, chicken, cat, dog and most preferably a human.

Said demethylating agent can be either a cytidine analog or a non-competitive inhibitor of methyltransferase as described above. Preferably, said demethylating agent is a cytidine analog.

Cytidine analogs with demethylating activity are well known from one of skill in the art. Examples of cytidine analogs with demethylating activity include 5-Azacytidine, 5,6-dihydro-5-azacytidine, 1-β-D-arabinofuranosyl-5-azacytidine, 5-Aza-2'-deoxycytidine (decitabine) or 1-(beta-D-ribofuranosyl)-1,2dihydropropyrimindin-2-one (zebularine).

Non-competitive inhibitors of methyltransferase are well known from one of skill in the art. Examples of non-competitive inhibitor of methyltransferase include 4-aminobenzoic acid derivatives like procanaimide and procaine.

Said medicament may also comprise an antigen.

An "antigen" as used herein is a molecule capable of inducing an immune response. The antigen may be any type of antigen known in the art and examples of antigens include but are not limited to peptides, polypeptides, cells, cell extracts, polysaccharides, polysaccharide conjugates, lipids, glycolipids and carbohydrates. The term "antigen" broadly includes any type of molecule, which is recognized by a host immune system as being foreign.

The antigen may be under a crude, purified or recombinant form and polypeptide/peptide antigens, including peptide mimics of polysaccharides, may also be encoded within nucleic acids.Said antigen can be a microbial or a tumor antigen. Preferably, said microbial antigen is a virus antigen.

A "microbial antigen" as used herein is an antigen of a microorganism and includes but is not limited to virus, bacteria, parasites and fungi. Preferably, said microbial antigen is a virus antigen. Such antigens include the intact microorganism as well as natural isolates and fragments or derivatives thereof and also synthetic compounds, which are identical to or similar to natural microorganism antigens and induce an immune response specific for that microorganism. Such synthetic compounds may be a peptide mimic of a polysaccharide antigen. A compound is similar to a natural microorganism if it induces an immune response against this natural microorganism antigen. Most such antigens are used routinely in the art and are well known to those of ordinary skill in the art.

Examples of infectious virus that have been found in humans include but are not limited to: Retroviridae (e.g., HIV), Picornaviridae (e.g., polio viruses, hepatitis A viruses, enteroviruses, human Cox-sackie viruses, rhinoviruses, echoviruses), Calciviridae (e.g., strains that cause gastroenteritis), Togaviridae (e.g., equine encephalitis viruses, rubella viruses), Flaviviridae (e.g., dengue viruses, encephalitis viruses, yellow fever viruses), Coronoviridae (e.g., coronaviruses), Rhabdoviridae (e.g., vesicular stomatitis viruses, rabies viruses), Filoviridae (e.g., ebola viruses), Paramyxoviridae (e.g., parainfluenza viruses, mumps viruses, measles viruses, respiratory syncytial viruses), Orthomyxoviridae (e.g., influenza viruses), Bungaviridae (e.g., Hantaan viruses, bunga viruses, phleboviruses and Nairo viruses), AArena viridae (hemorrhagic fever viruses), Reoviridae (e.g. reoviruses, orbiviviruses and rotaviruses), Birnaviridae, Hep-adnaviridae (Hepatitis B viruses), Parvoviridae (parvoviruses), Papovaviridae (papilloma viruses, polyoma viruses), Adenoviridae (most adenoviruses), Herpesviridae herpes simplex virus (HSV) 1 and 2, varicella zoster virus, cytomegalovirus (CMV), herpes virus, Poxviridae (variola viruses, vaccinia viruses, pox viruses), and Iridoviridae (e.g., African swine fever virus), and unclassified viruses (e.g., the agent of delta hepatitis B), the agents of non-A, non-B hepatitis (i.e. Hepatitis C), Norwalk and related viruses, and astroviruses).

Examples of bacteria that have been found in humans include both gram negative and gram positive bacteria. Such gram positive bacteria include, but are not limited to Pasteurella species, Staphylococci species, and Streptococcus species. Gram negative bacteria include, but are not limited to, *Escherichia coli,* Pseudomonas species, and Salmonella species. Specific examples of infectious bacteria include but are not limited to: Helicobacterpyloirs, Borelia burgdorferi, Legionella pneumophilia, Mycobacteria species (e.g., M. tuberculosis, M. avium, M. intracellulare, M. kansaii, M. gordonae), Staphylococcus aureus, Neisseria gornorrhoeae, Neisseria meningitides, Listeria monocytogenes, Streptococcus pyogenes (group A Streptococcus), *Streptococcus agalactia,* (group B Streptococcus), Streptococcus (viridans group), *Streptococcus faecalis, Streptococcus bovis,* Streptococcus (*anaerobic species*)*, Streptococcus pneumoniae,* pathogenic Campylobacter sp., Enterococcus sp., *Haemophilus influenzae, Bacillus antracis,* corynebacterium diphteriae, corynebacterium sp., Erysipelothrix rhusiopathiae, Clostridium perfringers, Clostridium tetani, Enterobacter aerogenes, *Klebsiella pneumoniae, Pastrurella multocida,* Bacteroides sp., *Fusobacterium nucleatum, Streptobacillus moniliformis, Treponema pallidium, Treponema pertenue, Leptospira, Rickettsia,* and *Actinomyces israelli.*

Examples of infectious fungi include, but are not limited to *Cryptococcus neoformans, Histoplasma capsulatum, Coccodioides imitis, Blastomyces dermatidis, Chlamydia trachomatis, Candida albicans.*

Examples of parasites include, but are not limited to Plasmodium such as *Plasmodium falciparum, Plasmodium malariae, Plasmodium ovale,* and *Plasmodium vivax.*

Other microorganisms include protists (e.g., *Toxoplama gondii*)*.* Other medically relevant microorganisms have been described in the literature, e.g., see THOMAS, Medical Microbiology, BAILLIERE TINDALL, GREAT BRITAIN, 1983, the entire content of which is hereby incorporated by reference.

Although many of the microbial antigens described above relate to human disorders, the invention is also useful for treating other non human vertebrates.

A "tumor antigen" as used herein is a compound, such as peptide, associated with a specific tumor or cancer cell surface and which is capable of inducing an immune response when expressed on the surface of an antigen presenting cell in the context of an MHC molecule. Tumor-specific antigens can be prepared from tumor cells, for example as described in COHEN et al. (Cancer Res., vol.54, p:1055, 1994) by partially purifying the antigens, by recombinant technology, or by *de novo* synthesis of known antigens.

Tumor-specific antigens are also well known from one of skill in the art. Examples of tumor-specific antigens include proteins specifically expressed in tumor or epitopes derived from such protein. Examples of protein specifically expressed in tumors include, but are not limited to MAGE A, BAGE, GAGE families and HER.

In some aspects of the invention the antigen is a polypeptide. Minor modifications of the primary amino acid sequences of polypeptide antigens may also result in a polypeptide, which has substantially equivalent antigenic activity as compared to the unmodified counterpart polypeptide. Such modifications may be deliberate, as by site direct mutagenesis, or may be spontaneous. All the polypeptides produced by these modifications are included herein as long as antigenicity still exists.

In a specific embodiment, the invention also utilizes polynucleotides encoding antigenic polypeptides. It is envisioned that the antigen may be delivered to the subject in a nucleic acid molecule which encodes for the antigen such that the antigen must be expressed in vivo. The nucleic acid encoding the antigen is operatively linked to a gene expression sequence which directs the expression of the antigen nucleic acid within a eukaryotic cell. The "gene expression sequence" is any regulatory nucleotide sequence, such as a promoter sequence or promoter-enhancer combination, which facilitates the efficient transcription and translation of the antigen nucleic acid to which it is operatively linked. The gene expression sequence may, for example, be a mammalian or viral promoter, such as a constitutive or inducible promoter. Constitutive mammalian promoters include, but are not limited to, the promoters for the following genes: hypoxanthine phosphoribosyl transferase (HPTR), adenosine deaminase, pyruvate kinase, .beta.-actin promoter, muscle creatine kinase promoter, human elongation factor promoter and other constitutive promoters. Exemplary viral promoters which function constitutively in eukaryotic cells include, for example, promoters from the simian virus (e.g., SV40), papilloma virus, adenovirus, human immunodeficiency virus (HIV), rous sarcoma virus, cytomegalovirus (CMV), Rous sarcoma virus (RSV), hepatitis B virus (HBV), the long terminal repeats (LTR) of Moloney leukemia virus and other retroviruses, and the thymidine kinase promoter of herpes simplex virus. Other constitutive promoters are known to those of ordinary skill in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Other inducible promoters are known to those of ordinary skill in the art.

In general, the gene expression sequence shall include, as necessary, 5' non-transcribing and 5' non-translating sequences involved with the initiation of transcription and translation, respectively, such as a TATA box, capping sequence, CAAT sequence, and the like. Especially, such 5' non-transcribing sequences will include a promoter region which includes a promoter sequence for transcriptional control of the operably joined antigen nucleic acid. The gene expression sequences optionally include enhancer sequences or upstream activator sequences as desired.

The antigen nucleic acid is operatively linked to the gene expression sequence. As used herein, the antigen nucleic acid sequence and the gene expression sequence are said to be "operably linked" when they are covalently linked in such a way as to place the expression or transcription and/or translation of the antigen coding sequence under the influence or control of the gene expression sequence. Two DNA sequences are said to be operably linked if induction of a promoter in the 5' gene expression sequence results in the transcription of the antigen sequence and if the nature of the linkage between the two DNA sequences does not (1) result in the introduction of a frame-shift mutation, (2) interfere with the ability of the promoter region to direct the transcription of the antigen sequence, or (3) interfere with the ability of the corresponding RNA transcript to be translated into a protein. Thus, a gene expression sequence would be operably linked to an antigen nucleic acid sequence if the gene expression sequence were capable of effecting transcription of that antigen nucleic acid sequence such that the resulting transcript is translated into the desired protein or polypeptide.

The « antigen nucleic acid sequence» may encode a protein, polypeptide, peptide, or peptide mimic of a polysaccbaride. It may also encode more than one antigenic component as a fusion construct. More than one antigen-encoding sequence may be included in the same plasmid vector and these may be linked to the same or different gene expression sequences.

The antigen nucleic acid may be delivered to the immune system alone or in association with a vector. In its broadest sense, a "vector" is any vehicle capable of facilitating the transfcr of the antigen nucleic acid to the cells of the immune system and preferably APCs so that the antigen can be expressed and presented on the surface of an APC. Preferably, the vector transports the nucleic acid to the immune cells with reduced degradation relative to the extent of degradation that would result in the absence of the vector. The vector optionally includes the above-described gene expression sequence to enhance expression of the antigen nucleic acid in APCs. In general, the vectors useful in the invention include, but are not limited to, plasmids, phagemids, viruses, other vehicles derived from viral or bacterial sources that have been manipulated by the insertion or incorporation of the antigen nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses, the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in KRIEGLER (A Laboratory Manual," W.H. Freeman C.O., New York, 1990) and in MURRY ("Methods in Molecular Biology," vol.7, Humana Press, Inc., Cliffton, N.J., 1991).

Preferred virus for certain applications are the adenovirus and adeno-associated virus which are double-stranded DNA viruses that have already been approved for human use in gene therapy and immunotherapy trials. The adeno-associated virus can be engineered to be replication deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well-known to those of skill in the art. See e.g., SANBROOK et al., "Molecular Cloning: A Laboratory Manual," Second Edition, Cold Spring Harbor Laboratory Press, 1989. In the last few years, plasmid vectors have been used as DNA vaccines for delivering antigen-encoding genes to cells *in vivo*. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUC19, pRC/CMV, SV40, and pBlueScript-. Other plasmids are well-known to those of ordinary skill in the art. Additionally, plasmids may be custom designed using restriction enzymes and ligation reactions to remove and add specific fragments of DNA. Plasmids such as those used for DNA vaccines may be delivered by a variety of parenteral, mucosal and topical routes. For example the plasmid DNA can be injected by intramuscular, intradermal, subcutaneous or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

Said medicament may also comprise an adjuvant like an adjuvant that creates a depo effect, an immune stimulating adjuvant, or an adjuvant that creates a depo effect and stimulates the immune system.

An "adjuvant that creates a depo effect" as used herein is an adjuvant that causes the antigen to be slowly released in the body, thus prolonging the exposure of immune cells to the antigen. Examples of such adjuvants are well known from one of skill in the art and include alum (e.g., aluminium hydroxide, aluminium phosphate) emulsion based formulations including mineral oil, non-mineral oil, water-in-oil or oil-in-water emulsions, such as the Seppic ISA series of MONTANIDE adjuvants (e.g., MONTANIDE ISA 720); MF-59 (CHIRON Corp, a squalene-in-water emulsion stabilized with SPAN 85 and TWEEN 80), and PROVAX (an oil-in-water emulsion containing a stabilizing detergent and a micelle-forming agent; IDEC, PHARMACEUTICALS Corp).

An "immune stimulating adjuvant" as used herein is an adjuvant that causes activation of a cell of the immune system. It may, for instance, cause an immune cell to produce and secrete cytokines. Examples of such adjuvants are well known from one of skill in the art and include CpG nucleotides, saponins purified from the bark of the *Q*. *saponaria* tree, such as QS21 (AQUILA BIOPHARMACEUTICALS Inc); poly[di(carboxylatophenoxy)phosphazene](PCPP polymer; VIRUS RESEARCH INSTITUTE) derivatives of polysaccharides such as monophosphoryl lipid A (MPL; RIBI IMMUNOCHEM RESEARCH Inc), muramyl dipeptide (MDP; RIBI) and threonyl-muramyl dipeptide (tMDP; RIBI); OM-174 (a glucosamine disaccharide related to lipid A; OM PHARMA SA); and Leishmania elongation factor (a purified Leishmania protein; CORIXA Corp).

An "adjuvant that create a depo effect and stimulate the immune system" as used herein is an adjuvant that have both of the above-identified functions. Examples of such adjuvants are well known from one of skill in the art and include ISCOMS (Immunostimulating complexes which contain mixed saponins, lipids and form virus-sized particles with pores that can hold antigen; CSL); SB-AS2 (SMITHKLINE BEECHAM ADLUVANT SYSTEM 2, which is an oil-in-water emulsion containing MPL and QS21, SBB); SB-AS4 (SMITHKLINE BEECHAM ADLUVANT SYSTEM 4, which contain alum and MPL; SBB); non-ionic block copolymers that form micelles such as CRL 1005 (these contain a linear chain of hydrophobic polyoxypropylene flanked by chains of polyoxyethylene; VAXCEL Inc); and SYNTEX Adjuvant Formulation (SAF, an oil-in-water emulsion containing TWEEN 80 and a non-ionic block copolymer; SYNTEX CHEMICALS Inc).

Said medicament may be adapted for various routes of administration. Examples of such administrations include orally, parenterally, intraperitoneally, intravenously, intraarterially, transdermally, sublingually, intramuscularly, rectally, transbucally, intranasally, liposomally, via inhalation, vaginally, intraoccularly, via local delivery (for example by catheter or stent), subcutaneously, intraadiposally, intraarticularly, or intrathecally.

Said medicament may also comprise a vehicle. For example, the medicament may comprise emulsions, microemulsions, oil-in-water emulsions, anhydrous lipids and oil-in-water emulsions, other types of emulsions. The medicament may also comprise one or more additives (e.g., diluents, excipients, stabilizers, preservatives). See, generally, Ullmann's Encyclopedia of Industrial Chemistry, 6th Ed. (various editors, 1989-1998, Marcel Dekker); and Pharmaceutical Dosage Forms and Drug Delivery Systems (ANSEL et al., 1994, WILLIAMS & WILKINS).

Amounts of the inventive combination of therapeutic or prophylactic agents can vary, according to determination made by one of skill in the art, but preferably are in amounts effective for inducing and enhancing the *in vivo* antigen-specific cytolysis by cytotoxic T lymphocytes (CTL). For the slow-release dosage form, appropriate release times can vary, but preferably should be last from 1 hour to about 6 months, most preferably from about 1 week to about 4 weeks. Formulations including the inventive combination of therapeutic or prophylactic agents and/or composition can vary, according to the particular situation, and as generally taught herein.

In a preferred embodiment, 5-azacytidine is administrated to a patient by injection. For example, 5-azacytidine is administrated by a daily injection of a dose ranging from 100-0.1 mg/kg of subject, preferably from 10-0.1 mg/kg and more preferably from 5-0.1 mg/kg, and from about 1 day to about 6 months, preferably from 3 days to 1 month, and more preferably from 1 week to 4 weeks.

5-azacytidine may be supplied as sterile powder for injection, together with buffering salt such as potassium dihydrogen and pH modifier such as sodium hydroxide. This formulation is preferably stored at 2-8°C, which should keep the drug stable for at least 2 years. This powder formulation may be reconstituted with sterile water for injection.

Said administration or coadministration may be done in any conventional dosage forms or in slow release dosage forms. Coadministration in the context of this invention is defined to mean the administration of more than one therapeutic or prophylactic in the course of a coordinated treatment to achieve an improved clinical outcome. Such coadministration may also be coextensive, that is, occurring during overlapping periods of times. For example, demethylating agent may be administrated to a subject after, concomitantly, or before the antigen. In a preferred embodiment, the subject may be pretreated with the antigen and then with the demethylating agent (e.g., a cytidine analog).

In a second aspect the present invention relates to a composition for inducing and enhancing antigen-specific *in vivo* cytolysis by cytotoxic T lymphocytes (CTL) comprising (i) a demethylating agent and (ii) a virus antigen.

Advantageously, said composition comprises effective amounts of a demethylating agent and of a virus antigen in order to induce and enhance the *in vivo* antigen-specific cytolysis by cytotoxic lymphocytes. Said effective amount are well known from one of skill in the art and depends of the used compounds.

Said demethylating agent can be either a cytidine analog or a non-competitive inhibitor of methyltransferase as described above. Preferably, said demethylating agent is a cytidine analog.

Said virus antigen is a virus antigen as described above.

Said virus antigen may be under a crude, purified or recombinant form and polypeptide/peptide antigens, including peptide mimics of polysaccharides, may also be encoded within nucleic acids.

In a specific embodiment, the invention also utilizes polynucleotides encoding antigenic polypeptides. It is envisioned that the virus antigen may be delivered to the subject in a nucleic acid molecule which encodes for the virus antigen as described above.

The composition may also comprise an adjuvant as described above.

Said composition may be adapted for various routes of administration as described above.

The composition may also comprise a vehicle and/or one or more additives as described above.

In a third aspect the present invention relates to a kit for inducing and enhancing *in vivo* antigen-specific cytolysis by cytotoxic T lymphocytes (CTL) comprising (i) a first composition comprising a demethylating agent, and (ii) a second composition comprising a virus antigen.

Said composition comprises an effective amount of demethylating agent and of virus antigen in order to induce and enhance the *in vivo* antigen-specific cytolysis by CTL as described above.

Advantageously, said demethylating agent of the first composition can be either a cytidine analog or a non-competitive inhibitor of methyltransferase as described above.

Said demethylating agent may be in a solid form, or solubilized in a buffer or water or incorporated in emulsions and microemulsions. Suitable buffers include, but are not limited to, phosphate buffered saline Ca⁺⁺/Mg⁺⁺ free (PBS), phosphate buffered saline (PBS), normal saline (150 mM NaCl in water), Tris buffer and surfactants.

Said antigen is a virus antigen as described above.

Said (i) first and (ii) second compositions may be adapted for various routes of administration as described above.

Said (i) first and (ii) second compositions may also comprise a vehicle and/or one or more additives as described above.

Advantageously, said (ii) composition may also comprise an adjuvant as described above.

### EXAMPLES

### MATERIALS AND METHODS

### In vivo experiments

Aldrithiol-2 (AT-2)-inactivated HIV (10⁹/50 µl) (LU and ANDRIEU, J. Virol., vol.75, p: 8949-56, 2001). or human chromogranin A (hCgA, a protein associated with neuroendocrine tumours (DEGORCE et al., Br. J. Cancer., vol.79, p:65-71, 1999); 10 µg/50 µl) plus incomplete Freund's adjuvant (IFA) (50 µl) were injected subcutaneously into 4- to 6-week-old male BALB/c mice (Institute of Laboratory Animal Science, Beijing, China). Mice immunized with AT-2-inactivated HIV (n = 12) or hCgA (n = 12) were randomly divided into two subgroups. One subgroup (n = 6) received daily intramuscular injection of 5-azacytidine (Sigma, St Louis, Missouri) at a dose of 5 mg/kg for 7 days and the other subgroup (n = 6) received 0.9% sodium chloride as control. Animals were killed at day 7 for ex vivo analysis. In addition, non-immunized mice (n = 6) injected with IFA alone were also killed on the same day to establish a background control.

### Ex vivo cytotoxic assay

Splenocytes were taken from 7-day-immunized mice and plated into Micro Tubes-Bulk (Bio-Rad, Hercules, CA) in the presence of CFSE (Molecular Probes, PoortGebouw, The Netherlands)-labelled AT-2-HIV- or hCgA-pulsed dendritic cells (DCs) at different E:T ratios for 4 h at 37°C. DCs generated from bone marrows of BALB/c mice (LUTZ et al., J. Immunol. Methods., vol.223, p: 77-92, 1999) were pulsed with AT-HIV (10⁹/ml) or hCgA (10 µg/ml) for 90 min. They were then labelled with CFSE (10 nM) for 15 min and washed twice. Non-pulsed DCs were labelled with CFSE as specificity control. At the end of the incubation, 10 µl of propidium iodide (PI, Sigma) (20 µg/ml) were added to each tube. Target cytolysis was analyzed on a FACSCalibur (BD Immunocytometry System, San Jose, CA). Virus- or tumour-specific cytolytic activity was determined by calculating the percentage of CFSE/PI-staining DCs after subtraction of the non-specific CFSE/PI-staining of non-pulsed DCs.

### In vivo cytotoxic assay

This assay was performed as described previously (AICHELE et al., Immunity, vol.6, p: 519-29, 1997). Briefly, target DCs were prepared from BALB/c bone marrow cell suspensions (LUTZ *et al.,* above mentioned, 1999). Cells were divided into two populations, one of which was pulsed with AT-2-HIV (10⁹/ml) or hCgA (10 µg/ml) for 90 min at 37°C; the control population was incubated in parallel in the absence of virus or tumour antigen. After the incubation, cells were labelled with CFSE at 1 µM (AT-2 HIV- or hCgA-pulsed DCs) or 100 nM (control population). The same number of cells (10⁷) from each population was mixed and injected intravenously into 7-day-immunized or non-immunized (control) BALB/c recipients. Spleen cells from recipients were collected 15 h after target DC injection and 10⁴ CFSE⁺ cells were acquired on the FACSCalibur. In vivo HIV- or hCgA-specific cytolysis was assessed by calculating the ratio of non-pulsed DCs/pulsed DCs.

### Statistical analysis

Impaired data between different groups of animals were compared by the MANN-WHITNEY analysis.

### RESULTS

### Enhanced ex vivo HIV-specific cytolysis in 5-Azacytidine-treated AT-2-HIV-immunized mice.

Ex vivo HIV-specific cytolysis of AT-2-HIV-pulsed DCs by splenocytes was increased >2 fold in 5-Azacytidine-treated immunized animals as compared with untreated immunized animals (P < 0.01) (Fig. 1A).

### Enhanced in vivo HIV-specific cytolysis in 5-Azacytidine-treated AT-2-HIV-immunized mice.

In vivo HIV-specific cytolysis of AT-2-HIV-pulsed DCs was increased >3 fold in 5-Azacytidine-treated immunized animals as compared with untreated immunized animals (P < 0.01) (Fig. 1B).

### Enhanced ex vivo HIV-specific cytolysis in 5-Azacytidine-treated hCgA-immunized mice.

Ex vivo hCgA-specific cytolysis of hCgA-pulsed DCs by splenocytes was increased >2 fold in 5-Azacytidine-treated immunized animals as compared with untreated immunized animals (P < 0.01) (Fig. 2A).

### Enhanced in vivo hCgA-specific cytolysis in 5-Azacytidine-treated hCgA-immunized mice.

In vivo hCgA-specific cytolysis of hCgA-pulsed DCs by splenocytes was increased >3 fold in 5-Azacytidine-treated immunized animals as compared with untreated immunized animals (P < 0.01) (Fig. 2B).

## Claims

1. Use of a demethylating agent for the manufacture of a medicament for the prevention or treatment of chronic viral diseases wherein antiviral CTL are present but not-functionally activated, wherein said demethylating agent induces a virus specific immunity and said medicament enhances the *in vivo* antigen-specific cytolysis by CTL in a subject.

2. Use according to claim 1, wherein said demethylating agent is:
- a cytidine analog with demethylating activity selected in the group comprising 5-Azacytidine, 5,6-dihydro-5-azacytidine, 1-β-D-arabinofuranosyl-5-azacytidine, 5-Aza-2'-deoxycytidine (decitabine) or 1-(beta-D-ribofuranosyl)-1,2dihydropropyrimindin-2-one (zebularine), or
- a non-competitive inhibitor of methyltransferase selected in the group comprising 4-aminobenzoic acid derivatives like procanaimide and procaine.

3. The use according to anyone of claims 1 or 2, wherein said medicament further comprises an antigen.

4. The use according to claim 3, wherein said antigen is a molecule capable of inducing an immune response selected in the group comprising peptides, polypeptides, cells, cell extracts, polysaccharides, polysaccharide conjugates, lipids, glycolipids and carbohydrates.

5. The use according to anyone of claims 3 or 4, wherein said antigen is a virus antigen.

6. The use according to anyone of claims 1 to 5, qherein said medicament further comprises an adjuvant.

7. The use according to anyone of claims 1 to 5, wherein said medicament further comprises a vehicle.

8. A composition for inducing and enhancing *in vivo* antigen-specific cytolysis by cytotoxic T lymphocytes (CTL) comprising a demethylating agent inducing a virus specific immunity and a virus antigen.

9. The product according to claim 8, wherein said demethylating agent is:
- a cytidine analog with demethylating activity selected in the group comprising 5-Azacytidine, 5,6-dihydro-5-azacytidine, 1-β-D-arabinofuranosyl-5-azacytidine, 5-Aza-2'-deoxycytidine (decitabine) or 1-(beta-D-ribofuranosyl)-1,2dihydropropyrimindin-2-one (zebularine), or
- a non-competitive inhibitor of methyltransferase selected in the group comprising 4-aminobenzoic acid derivatives like procanaimide and procaine.

10. The product according to anyone of claims 8 or 9, wherein said antigen is a molecule capable of inducing an immune response selected in the group comprising peptides, polypeptides, cells, cell extracts, polysaccharides, polysaccharide conjugates, lipids, glycolipids and carbohydrates.

11. The product according to anyone of claims 8 to 10, further comprising an adjuvant.

12. The product according to anyone of claims 8 to 11 further comprising a vehicle.

13. A kit for inducing and enhancing *in vivo* antigen-specific cytolysis by cytotoxic T lymphocytes (CTL) comprising (i) a first composition comprising a demethylating agent inducing a virus specific immunity, and (ii) a second composition comprising a virus antigen.

14. The kit according to claim 13, wherein said demethylating agent is:
- a cytidine analog with demethylating activity selected in the group comprising 5-Azacytidine, 5,6-dihydro-5-azacytidine, 1-β-D-arabinofuranosyl-5-azacytidine, 5-Aza-2'-deoxycytidine (decitabine) or 1-(beta-D-ribofuranosyl)-1,2dihydropropyrimindin-2-one (zebularine), or
- a non-competitive inhibitor of methyltransferase selected in the group comprising 4-aminobenzoic acid derivatives like procanaimide and procaine.

15. The kit according to anyone of claims 13 or 14, wherein said antigen is a molecule capable of inducing an immune response selected in the group comprising peptides, polypeptides, cells, cell extracts, polysaccharides, polysaccharide conjugates, lipids, glycolipids and carbohydrates.

16. The kit according to anyone of claims 13 to 15, wherein said (ii) second composition further comprises an adjuvant.

17. The kit according to anyone of claims 13 to 16, wherein said (i) first and/or (ii) second composition further comprises a vehicle.

## Patentansprüche

1. Verwendung eines Demethylierungsmittels zur Herstellung eines Arzneimittels zur Prävention oder Behandlung chronischer Viruserkrankungen, wobei antivirale ZTL vorhanden, jedoch nicht funktionell aktiviert sind, wobei das besagte Demethylierungsmittel eine virusspezifische Immunität induziert und das besagte Arzneimittel die antigenspezifische Zytolyse in vivo durch ZTL in einem Probanden verstärkt.

2. Verwendung nach Anspruch 1, wobei das besagte Demethylierungsmittel Folgendes ist:
ein Zytidin-Analogon mit Demethylierungsaktivität, das aus der Gruppe ausgewählt ist, die 5-Azazytidin, 5,6-Dihydro-5-azazytidin, 1-β-D-Arabinofuranosyl-5-azazytidin, 5-Aza-2'-desoxyzytidin (Decitabin) oder 1-(beta-D-Ribofuranosyl)-1,2-dihydropropyrimidin-2-on (Zebularin) umfasst, oder
ein nicht kompetitiver Inhibitor von Methyltransferase, der aus der Gruppe ausgewählt ist, die 4-Aminobenzoesäure-Derivate wie Procainamid und Procain umfasst.

3. Verwendung nach einem der Ansprüche 1 oder 2, wobei das besagte Arzneimittel weiterhin ein Antigen umfasst.

4. Verwendung nach Anspruch 3, wobei das besagte Antigen ein Molekül ist, das dazu in der Lage ist, eine Immunantwort zu induzieren, und aus der Gruppe ausgewählt ist, die Peptide, Polypeptide, Zellen, Zellextrakte, Polysaccharide, Polysaccharidkonjugate, Lipide, Glykolipide und Kohlenhydrate umfasst.

5. Verwendung nach einem der Ansprüche 3 oder 4, wobei das besagte Antigen ein Virusantigen ist.

6. Verwendung nach einem der Ansprüche 1 bis 5, wobei das besagte Arzneimittel weiterhin ein Hilfsmittel umfasst.

7. Verwendung nach einem der Ansprüche 1 bis 5, wobei das besagte Arzneimittel weiterhin einen Trägerstoff umfasst.

8. Zusammensetzung zum Induzieren und Verstärken antigenspezifischer Zytolyse in vivo durch zytotoxische T-Lymphozyten (ZTL), wobei die Zusammensetzung ein Demethylierungsmittel, das eine virusspezifische Immunität induziert, und ein Virusantigen umfasst.

9. Produkt nach Anspruch 8, wobei das besagte Demethylierungsmittel Folgendes ist:
ein Zytidin-Analogon mit Demethylierungsaktivität, das aus der Gruppe ausgewählt ist, die 5-Azazytidin, 5,6-Dihydro-5-azazytidin, 1-β-D-Arabinofuranosyl-5-azazytidin, 5-Aza-2'-desoxyzytidin (Decitabin) oder 1-(beta-D-Ribofuranosyl)-1,2-dihydropropyrimidin-2-on (Zebularin) umfasst, oder
ein nicht kompetitiver Inhibitor von Methyltransferase, der aus der Gruppe ausgewählt ist, die 4-Aminobenzoesäure-Derivate wie Procainamid und Procain umfasst.

10. Produkt nach einem der Ansprüche 8 oder 9, wobei das besagte Antigen ein Molekül ist, das dazu in der Lage ist, eine Immunantwort zu induzieren, und aus der Gruppe ausgewählt ist, die Peptide, Polypeptide, Zellen, Zellextrakte, Polysaccharide, Polysaccharidkonjugate, Lipide, Glykolipide und Kohlenhydrate umfasst.

11. Produkt nach einem der Ansprüche 8 bis 10, das weiterhin ein Hilfsmittel umfasst.

12. Produkt nach einem der Ansprüche 8 bis 11, das weiterhin einen Trägerstoff umfasst.

13. Kit zum Induzieren und Verstärken antigenspezifischer Zytolyse in vivo durch zytotoxische T-Lymphozyten (ZTL), wobei das Kit (i) eine erste Zusammensetzung, die ein Demethylierungsmittel, das eine virusspezifische Immunität induziert, umfasst, und (ii) eine zweite Zusammensetzung, die ein Virusantigen umfasst, umfasst.

14. Kit nach Anspruch 13, wobei das besagte Demethylierungsmittel Folgendes ist:
ein Zytidin-Analogon mit Demethylierungsaktivität, das aus der Gruppe ausgewählt ist, die 5-Azazytidin, 5,6-Dihydro-5-azazytidin, 1-β-D-Arabinofuranosyl-5-azazytidin, 5-Aza-2'-desoxyzytidin (Decitabin) oder 1-(beta-D-Ribofuranosyl)-1,2-dihydropropyrimidin-2-on (Zebularin) umfasst, oder
ein nicht kompetitiver Inhibitor von Methyltransferase, der aus der Gruppe ausgewählt ist, die 4-Aminobenzoesäure-Derivate wie Procainamid und Procain umfasst.

15. Kit nach einem der Ansprüche 13 oder 14, wobei das besagte Antigen ein Molekül ist, das dazu in der Lage ist, eine Immunantwort zu induzieren, und aus der Gruppe ausgewählt ist, die Peptide, Polypeptide, Zellen, Zellextrakte, Polysaccharide, Polysaccharidkonjugate, Lipide, Glykolipide und Kohlenhydrate umfasst.

16. Kit nach einem der Ansprüche 13 bis 15, wobei die besagte (ii) zweite Zusammensetzung weiterhin ein Hilfsmittel umfasst.

17. Kit nach einem der Ansprüche 13 bis 16, wobei die besagte (i) erste und/oder (ii) zweite Zusammensetzung weiterhin einen Trägerstoff umfasst.

## Revendications

1. Utilisation d'un agent de déméthylation pour la fabrication d'un médicament destiné à la prévention ou au traitement de maladies virales chroniques dans lesquelles des CTL antiviraux sont présents mais ne sont pas activés fonctionnellement, dans laquelle ledit agent de déméthylation induit une immunité spécifique contre les virus et ledit médicament renforce la cytolyse spécifique d'un antigène in vivo par les CTL chez un sujet.

2. Utilisation selon la revendication 1, dans laquelle ledit agent de déméthylation est :
un analogue de cytidine ayant une activité de déméthylation choisi dans le groupe comprenant la 5-azacytidine, la 5,6-dihydro-5-azacytidine, la 1-β-D-arabinofuranosyl-5-azacytidine, la 5-aza-2'-désoxycytidine (décitabine) ou la 1-(bêta-D-ribofuranosyl)-1,2-dihydropropyrimindin-2-one (zébularine), ou
un inhibiteur non compétitif de méthyltransférase choisi dans le groupe comprenant des dérivés de l'acide 4-aminobenzoïque comme le procanaïmide et la procaïne.

3. Utilisation selon l'une quelconque des revendications 1 ou 2, dans laquelle ledit médicament comprend en outre un antigène.

4. Utilisation selon la revendication 3, dans laquelle ledit antigène est une molécule capable d'induire une réponse immunitaire choisie dans le groupe comprenant des peptides, des polypeptides, des cellules, des extraits cellulaires, des polysaccharides, des conjugués de polysaccharide, des lipides, des glycolipides et des carbohydrates.

5. Utilisation selon la revendication 3 ou 4, dans laquelle ledit antigène est un antigène de virus.

6. Utilisation selon l'un quelconque des revendications 1 à 5, dans laquelle ledit médicament comprend en outre un adjuvant.

7. Utilisation selon l'un quelconque des revendications 1 à 5, dans laquelle ledit médicament comprend en outre un véhicule.

8. Composition pour induire et renforcer une cytolyse spécifique d'un antigène in vivo par les lymphocytes T cytotoxiques (CTL) comprenant un agent de déméthylation induisant une immunité spécifique contre les virus et un antigène de virus.

9. Produit selon la revendication 8, dans lequel ledit agent de déméthylation est :
un analogue de cytidine ayant une activité de déméthylation choisi dans le groupe comprenant la 5-azacytidine, la 5,6-dihydro-5-azacytidine, la 1-β-D-arabinofuranosyl-5-azacytidine, la 5-aza-2'-désoxycytidine (décitabine) ou la 1-(bêta-D-ribofuranosyl)-1,2-dihydropropyrimindin-2-one (zébularine), ou
un inhibiteur non compétitif de méthyltransférase choisi dans le groupe comprenant des dérivés de l'acide 4-aminobenzoïque comme le procanaïmide et la procaïne.

10. Produit selon l'une quelconque desrevendications 8 ou 9, dans lequel ledit antigène est une molécule capable d'induire une réponse immunitaire choisie dans le groupe comprenant des peptides, des polypeptides, des cellules, des extraits cellulaires, des polysaccharides, des conjugués de polysaccharide, des lipides, des glycolipides et des glucides.

11. Produit selon l'une quelconque des revendications 8 à 10, comprenant en outre un adjuvant.

12. Produit selon l'une quelconque des revendications 8 à 11, comprenant en outre un véhicule.

13. Kit pour induire et renforcer une cytolyse spécifique d'un antigène in vivo par les lymphocytes T cytotoxiques (CTL) comprenant (i) une première composition comprenant un agent de déméthylation induisant une immunité spécifique contre les virus et (ii) une seconde composition comprenant un antigène de virus.

14. Kit selon la revendication 13, dans lequel ledit agent de déméthylation est :
un analogue de cytidine ayant une activité de déméthylation choisi dans le groupe comprenant la 5-azacytidine, la 5,6-dihydro-5-azacytidine, la 1-□-D-arabinofuranosyl-5-azacytidine, la 5-aza-2'-désoxycytidine (décitabine) ou la 1-(bêta-D-ribofuranosyl)-1,2-dihydropropyrimindin-2-one (zébularine), ou
un inhibiteur non compétitif de méthyltransférase choisi dans le groupe comprenant des dérivés de l'acide 4-aminobenzoïque comme le procanaïmide et la procaïne.

15. Kit selon l'une quelconque des revendications 13 ou 14, dans lequel ledit antigène est une molécule capable d'induire une réponse immunitaire choisie dans le groupe comprenant des peptides, des polypeptides, des cellules, des extraits cellulaires, des polysaccharides, des conjugués de polysaccharide, des lipides, des glycolipides et des carbohydrates.

16. Kit selon l'une quelconque des revendications 13 à 15, dans lequel ladite (ii) seconde composition comprend en outre un adjuvant.

17. Kit selon l'une quelconque des revendications 13 à 16, dans lequel ladite (i) première composition et/ou (ii) seconde composition comprend en outre un véhicule.
